# DEMANDE DE BREVET EUROPEEN

(11) **EP 0 759 423 A1**
(43) Date de publication de la demande: **26.02.1997**
(21) Numéro de dépôt: 96401757.8
(22) Date de dépôt: 08.08.1996
(51) Int. Cl.: C07C 67/04, C07C 69/54

(54) **Procédé de préparation de (méth)acrylate d'isobornyle**

(30) Priorité: 18.08.1995 FR 9509916
(71) Demandeur: ELF ATOCHEM S.A., 92800 Puteaux (FR)
(72) Inventeur: Riondel, Alain, 57600 Forbach (FR)
(74) Mandataire: Rieux, Michel

(57) **Abrégé**

Ce procédé de préparation de (méth)acrylate d'isobornyle consiste à faire réagir l'acide (méth)acrylique sur le camphène en présence d'un superacide solide à base de zirconium comme catalyseur.

## Description

La présente invention est relative à un procédé de préparation du méthacrylate d'isobornyle et de l'acrylate d'isobornyle.

Le brevet des Etats-Unis d'Amérique N°3 087 962 propose un procédé de préparation de l'acrylate ou du méthacrylate d'isobornyle par réaction de l'acide acrylique ou méthacrylique sur le camphène en notant qu'il se produit alors un réarrangement. Le procédé est effectué en présence d'un catalyseur acide fort, tel que l'acide sulfurique, ou un acide de Lewis, tel que le trifluorure de bore. Ce procédé ne peut pas être utilisé à l'échelle industrielle en raison de son faible rendement et de la corrosion des réacteurs provoquée par le trifluorure de bore.

Pour y remédier, la demande de brevet japonais publiée sous le N°58 049 337 propose un procédé de préparation de l'acrylate et du méthacrylate d'isobornyle du même type que celui rappelé ci-dessus, mais dans lequel la réaction est catalysée par une résine cationique forte sulfonique. Il se produit des réactions secondaires qui ont pour origine principale la dimérisation du camphène en diterpène, ce qui porte atteinte à la sélectivité de la réaction.

L'invention pallie ces inconvénients en utilisant un catalyseur qui donne une sélectivité en acrylate et méthacrylate d'isobornyle meilleure que tous les catalyseurs connus et qui représente le meilleur compromis rendement et sélectivité en ester.

L'invention a donc pour objet un procédé de préparation de l'acrylate et du méthacrylate d'isobornyle de formule 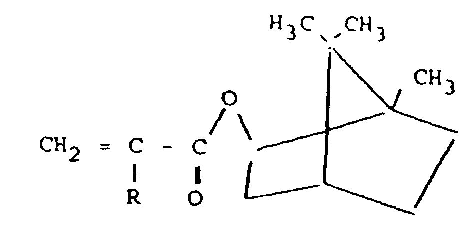 dans laquelle R est un atome d'hydrogène ou un radical méthyle par réaction de l'acide acrylique ou méthacrylique de formule 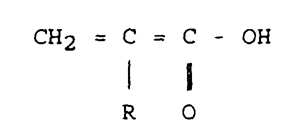 sur le camphène de formule 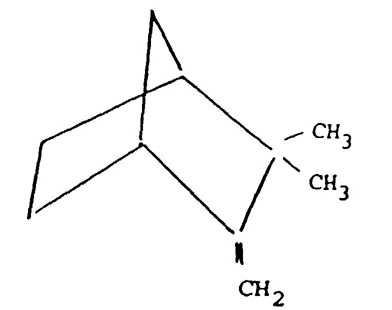 en présence d'une quantité catalytique d'un catalyseur acide, caractérisé en ce qu'il consiste à utiliser, comme catalyseur, un superacide solide à base de zirconium.

Le rapport molaire de l'acide au camphène peut être compris entre 4/1 et 1/4, et de préférence entre 2/1 et 1/2.

La température de réaction est comprise entre 10 et 60°C, et de préférence entre 30 et 40°C.

La durée de réaction peut être comprise entre 2 et 10 heures.

L'utilisation de solvants, tels que le cyclohexane, l'hexane, le toluène, est possible.

Il est recommandé d'utiliser sous bullage d'air des inhibiteurs, tels que l'hydroquinone, l'éther monométhylique de l'hydroquinone, des phénols substitués par des groupements alkyles à fort encombrement stérique, la phénothiazine, en quantité comprise entre 150 et 3000 ppm par rapport à l'acide.

Comme catalyseur, on peut utiliser en quantité comprise entre 2 et 20% en poids par rapport à la charge totale et de préférence 5 à 15% notamment le produit de réaction de l'hydroxyde de zirconium et du sulfate d'ammonium que l'on peut préparer de la manière suivante :

I - Préparation du Zr(OH)₄
dans un réacteur d'1 litre double enveloppe, muni de :
- une agitation
- une sonde de température
- une sonde pH on dissout à température ambiante 88 g de ZrOCl₂ octahydraté dans 800 ml d'eau.

On introduit lentement, toujours à température ambiante, environ 50 ml de NH₄OH à 25 % de telle façon que le pH de la solution finale soit compris entre 8 et 9.

Le précipité blanc de Zr(OH)₄ obtenu est ensuite :
1) lavé à l'eau pour éliminer les ions chlorures
2) séché à l'étuve à 100°C sous pression réduite pendant une nuit.

On obtient ainsi environ 40 g de Zr(OH)₄ sec.

Il - Sulfatation - calcination

On mélange à l'aide d'un broyeur Zr(OH)₄ et NH₄(SO₄)₂ (15 % en poids/Zr(oH)₄).

Le solide obtenu est ensuite calciné sous air sec à 650°C.

Après refroidissement, on obtient un solide blanc que l'on désigne par Zr SA 15.

### Exemple 1 Préparation du méthacrylate d'isobornyle (MAISOBOR)

Dans un réacteur en verre double enveloppe de 250 ml muni d'une sonde de température, on introduit le catalyseur ZrSA 15 (20,5 g), l'éther méthylique de l'hydroquinone (0,018 g) et une partie de l'acide méthacrylique (49,5 g). L'ensemble est porté sous bullage d'air à 35°C et on introduit dans le milieu en 1,5 H le camphène (136 g) dissout dans l'acide méthacrylique restant (43,8 g). Le milieu est ensuite maintenu 4,5 heures sous agitation. En fin de réaction un échantillon du brut réactionnel est analysé en:
- potentiométrie
- chromatographie en phase gazeuse pour déterminer la conversion du camphène, le rendement en méthacrylate d'isobornyle/camphène, la sélectivité en méthacrylate d'isobornyle/camphène.

Les résultats obtenus sont rassemblés dans le tableau 1.

### Exemple 2 (comparatif)

On reprend l'exemple 1, si ce n'est que l'on remplace le catalyseur par une résine acide comportant des groupes sulfoniques Amberlyst 15 (A15).

Les résultats obtenus sont consignés dans le tableau 1:

**Tableau 1**

| Synthèse du MAISOBOR Résultats | | | |
|---|---|---|---|
| | C(%) Conversion camphène | R(%) en MAISOBOR | S(%) Selectivité en MAISOBOR |
| Exemple 1 | 74,14 | 73,4 | 98,9 |
| Exemple 2 | 86 | 75 | 87,8 |

Dans l'exemple 2 la conversion est meilleure, mais on a davantage de sous produits.

Il ressort de ces essais que le meilleur compromis rendement-sélectivité est obtenu en utilisant ZrSA15.

### Exemple 3 Préparation de l'acrylate d'isobornyle (AISOBOR)

On reprend les conditions opératoires de l'exemple 1, si ce n'est que l'on remplace l'acide méthacrylique par l'acide acrylique. La quantité d'acide acrylique engagée est de 75,5 g, dont 47,5 g sont introduits dès le départ dans le réacteur. En fin de réaction, un échantillon du brut réactionnel est analysé de la même façon que lors de l'exemple 1, pour déterminer la conversion du camphène, le rendement en acrylate d'isobornyle/camphène, la sélectivité en acrylate d'isobornyle/camphène. Les résultats obtenus sont donnés dans le tableau 2.

### Exemple 4

On reprend l'exemple 3, si ce n'est que l'on remplace le catalyseur par une résine acide comportant des groupes sulfoniques Amberlyst 15 (A15).

Les résultats obtenus sont rapportés dans le tableau 2.

**Tableau 2**

| Synthèse de l'AISOBOR. Résultats | | | |
|---|---|---|---|
| Conversion du camphène (%) | | Rendement en AISOBOR (%) | Selectivité AISOBOR (%) |
| Exemple 3 | 75,1 | 73,8 | 98,2 |
| Exemple 4 | 88,2 | 84,7 | 96,2 |

Sur le plan sélectivité, on constate que ZrSA15 est sensiblement meilleur que A15.

## Revendications

1. procédé de préparation de (méth)acrylate d'isobornyle de formule 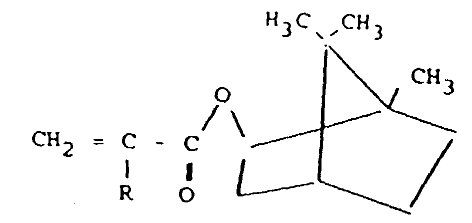 dans laquelle R est un atome d'hydrogène ou un radical méthyle par réaction de l'acide (méth)acrylique de formule 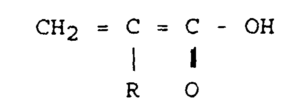 sur le camphène de formule 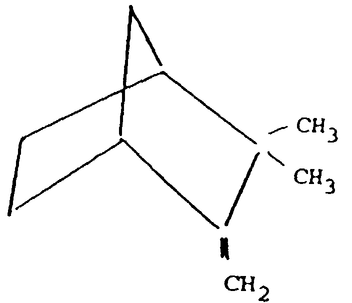 en présence d'une quantité catalytique d'un catalyseur acide, caractérisé en ce qu'il consiste à utiliser, comme catalyseur, un superacide solide à base de zirconium.

2. Procédé suivant la revendication 1, caractérisé en ce qu'il consiste à utiliser comme catalyseur le produit de réaction de l'hydroxyde de zirconium et du sulfate d'ammonium.

3. Procédé suivant la revendication 1 ou 2, caractérisé en ce que le rapport molaire de l'acide au camphène est compris entre 4/1 et 1/4, et de préférence entre 2/1 et 1/2.

4. Procédé suivant l'une des revendications précédentes, caractérisé en ce qu'il consiste à mettre le mélange en contact avec le catalyseur à une température comprise entre 10 et 60°C, et de préférence entre 30 et 40°C.
